(19) Europäisches Patentamt
European Patent Office
Office européen des brevets

(11) Veröffentlichungsnummer : **0 068 356**
**B1**

# (12) EUROPÄISCHE PATENTSCHRIFT

(45) Veröffentlichungstag der Patentschrift : **10.09.86**

(51) Int. Cl.⁴ : **C 12 Q 1/28**

(21) Anmeldenummer : **82105365.9**

(22) Anmeldetag : **18.06.82**

## (54) Mittel und Verfahren zum Nachweis von Wasserstoffperoxid oder Peroxidasen.

(30) Priorität : **23.06.81 DE 3124594**

(43) Veröffentlichungstag der Anmeldung : **05.01.83 Patentblatt 83/01**

(45) Bekanntmachung des Hinweises auf die Patenterteilung : **10.09.86 Patentblatt 86/37**

(84) Benannte Vertragsstaaten : **AT BE CH DE FR GB IT LI LU NL SE**

(56) Entgegenhaltungen :
**EP-A- 0 007 787**
**DD-A- 140 175**
**DE-A- 3 037 342**
**DE-B- 2 833 612**
**Chemical Abstracts Band 95, Nr. 5 3. August 1981 Columbus, Ohio, USA EIKEN CHEMICAL CO. LTD: "Spectrometric determination of hydrogen peroxide" Seite 371, Spalte 2, Abstract Nr. 38670t**
**Japanische Offenlegungsschrift 37 656/81**

(73) Patentinhaber : **Boehringer Mannheim GmbH**
**Sandhofer Strasse 116**
**D-6800 Mannheim 31 (DE)**

(72) Erfinder : **Träger, Ulrich, Dr.rer.nat.**
**Eschkopfstrasse 6**
**D-6703 Limburgerhof (DE)**
Erfinder : **Sojka, Bernward, Dr.rer.nat.**
**Bunsenstrasse 4**
**D-6806 Viernheim (DE)**
Erfinder : **Lange, Hans**
**Danzigerstrasse 3**
**D-6840 Lampertheim (DE)**

EP 0 068 356 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Mittel und Verfahren zum Nachweis von Wasserstoffperoxid oder Peroxidasen durch oxidative Kupplung von Chromogenen.

Die analytische Bestimmung von Wasserstoffperoxid hat für die medizinische Diagnostik eine erhebliche Bedeutung, da bei einer großen Anzahl von wichtigen Nachweisverfahren Wasserstoffperoxid als Zwischenprodukt gebildet wird, welches danach durch Umsetzung mit geeigneten chromogenen Substanzen, überwiegend in Gegenwart einer Peroxidase (POD) als Katalysator, in eine optisch erfaßbare Substanz überführt wird. Das Ausmaß der Bildung des optisch erfaßbaren Produktes dient als Maß für die Menge des Wasserstoffperoxids bzw. als Maß für die Menge des Wasserstoffperoxid-bildenden Substrates.

In Umkehrung dieser Reaktion kann unter Zusatz von $H_2O_2$ oder einer $H_2O_2$-bildenden Substanz auch die Konzentration von Peroxidasen oder peroxidatisch wirksamen Verbindungen, wie Hämoglobin, bestimmt werden.

Für diese Reaktion sind zahlreiche Chromogene bzw. Indikatorsysteme vorgeschlagen worden. Eines der am häufigsten angewandten Indikatorsysteme ist das von Trinder (Ann. Clin. Biochem. 6 (1969) 24-27), bei dem Phenol mit 4-Aminoantipyrin in Gegenwart von POD unter Einwirkung von $H_2O_2$ oxidativ zu einem Farbstoff gekuppelt wird. Anstelle des Phenols können auch Phenolderivate, Anilinderivate, Naphthol, Naphtholderivate, Naphthylamin, Naphthylaminderivate oder ähnlich reagierende Substanzen eingesetzt werden. Anstelle des 4-Aminoantipyrins können auch andere Aminoantipyrinderivate, Vanillindiaminsulfonsäure, Methylbenzothiazolonhydrazon (MBTH), sulfoniertes Methylbenzothiazolonhydrazon (S-MBTH) und ähnlich reagierende Verbindungen eingesetzt werden.

Als besonders empfindliches Nachweissystem wird gemäß DE-AS 28 33 612 die Kondensation von 4-Aminoantipyrin mittels N,N-Dialkyl- bzw. N-Hydroxyalkylsubstituiertem 3-Alkylanilin beschrieben. Die entstehenden Kupplungsprodukte weisen einen hohen molaren Extinktionskoeffizienten auf, so daß sie für den Nachweis geringer Mengen von Wasserstoffperoxid bzw. entsprechenden Wasserstoffperoxidbildenden Verbindungen gut geeignet sind. Wie in der vorstehenden Anmeldung gezeigt wurde, sind die durch Kupplung mit 4-Aminoantipyrin erzeugten Farbstoffe innerhalb normaler Meßzeiten stabil, jedoch zeigt sich, daß die Anilinderivate selbst, insbesondere das bevorzugte N-Ethyl-N-hydroxyethyl-3-methylanilin (EHT) nicht genügend lagerstabil sind und sich, insbesondere bei Imprägnierung auf Teststreifen, innerhalb einiger Wochen zersetzen. Darüberhinaus sind diese Verbindungen bei Raumtemperatur flüssig, so daß sir für Imprägnierungen auf Reagenträgern ebenfalls nicht sehr günstig sind. Da es sich um schwache Basen handelt, kann man ihre kristallinen Salze mit Mineralsäuren bei pH-Werten von unter 6 einsetzen, nicht jedoch in dem für diagnostische Zwecke besonders interessanten Gebiet zwischen pH 6 und 8.

Die Kupplung der N-Alkyl-N-hydroxyalkylaniline mit Benzothiazolinonhydrazonen, insbesondere 2-Hydrazono-2,3-dihydro-3-methylbenzothiazol-sulfonsäure-6 (S-MBTH) ergibt Farbstoffe mit einer besonders hohen Extinktion, so daß S-MBTH für den Nachweis geringer Mengen $H_2O_2$ oder POD an sich noch vorteilhafter eingesetzt werden könnte als 4-Aminoantipyrin. Es hat sich jedoch gezeigt, daß die mit EHT gewonnenen Farbstoffe instabil sind. Insbesondere treten Überoxidationen und andere Nebenwirkungen ein, die zu einer Veränderung des Absorptionsspektrums führen.

Es stellte sich deshalb die Aufgabe, eine Kupplungskomponente für eine oxidative Kupplung zu finden, die auch bei längerer Lagerung in fein verteiltem Zustand, beispielsweise bei Imprägnierung auf einem Reagensträger und in der Reagenslösung, stabil ist, bei Raumtemperatur in einem pH-Bereich von 6-8 fest ist, bei der oxidativen Kupplung Farbstoffe mit hoher molarer Extinktion ergibt, die zumindest für einen Beobachtungszeitraum von 1 Stunde farbstabil sind, so daß die Farbtiefe als Maß für die Menge des nachzuweisenden Wasserstoffperoxids dienen kann.

Diese Aufgabe wird erfindungsgemäß dadurch gelöst, daß man als kupplungsfähiges Substrat eine Verbindung der allgemeinen Formel I

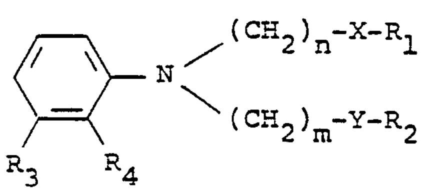

(I)

in der

n und m eine ganze Zahl von 1-4,

X und Y einen Valenzstrich oder einen Phenylenrest,

$R_1$ und $R_2$ eine Carboxyl-, oder Sulfonsäuegruppe darstellen, wobei eine der beiden Gruppen auch Wasserstoff oder eine niedere Alkylgruppe sein kann und

$R_3$ und $R_4$ Wasserstoff oder eine Alkylgruppe mit 1-6 vorzugsweise 1-2 C-Atomen darstellt sowie ihre Salze und als oxidationsfähige Substanz 2-Hydrazono-2,3-dihydro-3-methylbenzthiazol-sulfosäure-6 (S-MBTH) enthalten sind oder daß man als kupplungsfähiges Substrat eine Verbindung der allgemeinen Formel I

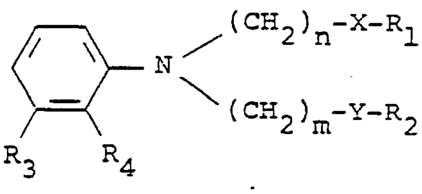

in der

n und m eine ganze Zahl von 1-4,

X und Y einen Valenzstrich oder einen Phenylenrest,

$R_1$ und $R_2$ eine Carboxyl- oder Sulfonsäuregruppe darstellen, wobei eine der beiden Gruppen auch Wasserstoff oder eine niedere Alkylgruppe sein kann und

$R_3$ und $R_4$ Wasserstoff oder eine Alkylgruppe mit 1-6, vorzugsweise 1-2 C-Atomen darstellt sowie ihre Salze, wobei $R_3$ eine Alkylgruppe darstellt wenn X-$R_1$ oder Y-$R_2$ eine Sulfo-Phenylengruppe darstellt, einsetzt.

Als oxidationsfähige Komponente werden 4-Aminoantipyrin (4-AAP), 2-Hydrazono-2,3-dihydro-3-methylbenzthiazol (MBTH) und insbesondere die 2-Hydrazono-2,3-dihydro-3-methylbenzthiazol-sulfonsäure-6 (S-MBTH) bevorzugt, jedoch können auch andere für ihre oxidative Kupplungsfähigkeit bekannte Substanzen in gleicher Weise eingesetzt werden.

Die erfindungsgemäßen Anilinderivate enthalten eine saure und eine basische Gruppe und liegen deshalb normalerweise als innere Salze vor. Ihre Löslichkeit und Handhabbarkeit läßt sich jedoch noch weiter verbessern, wenn man sie in Form ihrer Salze, insbesondere der Alkali- oder Ammoniumsalze einsetzt. Aufgrund der erhöhten Extinktion sind diejenigen Verbindungen bevorzugt, die in der 3-Stellung zum Aminstickstoff eine Alkylgruppe, insbesondere eine Methylgruppe enthalten.

Diese Anilinderivate sind zum Teil bekannte Verbindungen (vgl. J. Am. Chem. Soc. *78*, 5 827 (1956)) oder können in analoger Weise zu den bekannten Verbindungen hergestellt werden.

Wie bereits vorstehend gesagt, dient das erfindungsgemäße System zum Nachweis von Peroxidasen oder $H_2O_2$ bzw. Wasserstoffperoxid-produzierenden Systemen. Unter Wasserstoffperoxid-produzierenden Systemen sind insbesondere die in der klinischen Diagnostik wichtigen Substrat-/Substratoxidasepaare zu verstehen, bei denen das Substrat in Gegenwart von Luftsauerstoff oxidiert und $H_2O_2$ produziert wird. Je nachdem, welche der beiden Komponenten dem Reagens zugesetzt wird, kann entweder das Substrat oder die Substratoxidase nachgewiesen werden. Beispielhaft seien die Systeme Glukose/Glukoseoxidase, Cholesterin/Cholesterinoxidase, Harnsäure/Uricase, Creatinin/Creatininase und Sarkosinoxidase genannt. Die erfindungsgemäßen Reagenskombinationen bestehen demgemäß aus dem Substrat bzw. der Substratoxidase, Peroxidase, dem oxidationsfähigen Chromogen sowie einer der erfindungsgemäßen Substanzen der Formel I sowie gegebenenfalls Puffern, Netzmitteln, Stabilisierungsmitteln, Aktivatoren etc. entweder in Lösung oder in einem geeigneten festen Träger, vorzugsweise einem saugfähigen Papier. Mengen und Zusammensetzung können vom Fachmann aufgrund der bekannten ähnlichen Teste ohne weiteres bestimmt werden, wobei die folgenden Beispiele als Anhalt dienen können, ohne daß die Erfindung darauf beschränkt ist.

## Beispiel 1

Bestimmung von $H_2O_2$ in Lösungen

Reagenzlösungen

| Lösung A | Lösung B |
|---|---|
| 0,2 mmol MBTHS*/l | 0,2 mmol 4-Aminoantipyrin/l |
| 1 mmol Substrat/l | 1 mmol Substrat/l |
| 200 U Peroxidase/l | 200 U Peroxidase/l |
| 0,1 mol Phosphatpuffer/l (pH 7,0) | 0,1 mol Phosphatpuffer/l (pH 7,0) |
| 0,5 % Detergenz (Fettalkohol-polyglykolether) | 0,5 % Detergenz |

* 2-Hydrazino-2,3-dihydro-3-methylbenzthiazol-6-sulfosäure

Je 2 ml dieser Lösungen werden mit 0,05 ml einer 2 m molaren $H_2O_2$-Lösung versetzt nach ca. 60

Sekunden gut gemischt und die Extinktion in 1 cm Küvetten in einem Photometer nach 5-15 Minuten gemesen.

Folgende Extinktionen wurden ermittelt :

| Substrat | MBTHS | | | 4-Aminoantipyrin | | |
|---|---|---|---|---|---|---|
| | Extinktion in mE | | | Extinktion in mE | | |
| | λ | 5 min | 15 min | λ | 5 min | 15 min |
| N,N-Di-(hydroxyethyl)-m. toluidin (DHT) | 585 | 780 | 650 | - | - | - |
| N-Ethyl-N-hydroxyethyl-m. toluidin (EHT) | 580 | 660 | 560 | - | - | - |
| N,N-Di-(carboxylatoethyl-dinatriumsalz)-m. toluidin (DCET) | 585 | 753 | 755 | 550 | 424 | 419 |
| N-Ethyl-N-sulfoethyl-m. toluidin (EST) | 590 | 874 | 873 | 550 | 487 | 483 |
| N-Ethyl-N-sulfophenylenmethyl-m. toluidin (ESPT) | 600 | 760 | 758 | 550 | 432 | 425 |
| N-Ethyl-N-carboxylatophenylen-methyl-m. toluidin-natrium-salz (ECPT) | 600 | 738 | 735 | 550 | 463 | 455 |
| N-Ethyl-N-sulfophenylenmethyl-anilin (ESPA) | 600 | 750 | 747 | - | - | - |

## Beispiel 2

Bestimmung von Harnsäure im Serum

In einem geeigneten Glasgefäß, in welchem 80 ml einer 1 Jahr bei 20 °C stabilen 0,1 molaren Kaliumphosphat-Pufferlösung pH 7,0, welche zusätzlich 0,5 % Detergenz und 1 mmol/1 N-Ethyl-N-(2-sulfophenylenmethyl)-m-toluidin (ESPT) enthält, vorgelegt ist, wird ein Reagenzstreifen von ca. 120 mm Länge und 10 mm Breite eingestellt, der in 3 Bezirken je 2 überreinandergelegte Reagenzträger der Fläche 10 × 15 mm in eingenetzter Form enthält. Die beiden am unteren Ende des Reagenzstreifens angeklemmten Bezirke enthalten pro Reagenzträger 2,5 mg-Methylbenzthiazolonhydrazon-6-Sulfonsäure (MBTHS) zusammen mit je 1,7 mg $K_4[Fe(CN)_6]$ und darüberliegend je 1 Reagenzträger mit 6 Einheiten Uricase.

Der dritte Reagenzbezirk enthält 2 Reagenzträger mit je 6 Einheiten Uricase, 3 mg Tris/Citratpuffer pH 7 und 20 Einheiten Peroxidase (POD). Der Reagenzstreifen wird 10 Sekunden intensiv bewegt, anschließend wird 5 Minuten stehen gelassen, nochmals 10 Sekunden bewegt und der eluierte Reagenzträger verworfen.

Es entsteht eine Reaktionslösung mit folgender Zusammensetzung :

| | |
|---|---|
| Kaliumphosphat-Puffer pH 7,0 | 0,1 mol/l |
| ESPT | 1 mmol/l |
| Detergenz (Fettalkohol-polyglykolether) | 0,5 % |
| MBTHS | 0,2 mmol/l |
| $K_4[Fe(CN)_6]$ | 20 mg/l |
| Uricase | 255 U/l |
| POD | 470 U/l |

Der Harnsäuregehalt im Serum wird ermittelt, indem zu 2 ml dieser Reaktionslösung 0,050 ml Probe hinzugegeben und nach 20 Minuten Inkubation bei 20-25 °C die Extinktion bei Hg 578 nm oder an einem Spektralphotometer bei 590 nm gegen einen Standard ermittelt wird.

## Beispiel 3

Bestimmung von Creatinin im Serum

Testbestandteile :

a) Pufferlösung :

| | |
|---|---|
| Phosphatpuffer pH 7,5 | 0,1 mol/l |
| N-Ethyl-N-(2-sulfoethyl)-m-toluidin (EST) | 1 mmol/l |
| Detergenz | 0,5 % |

b) Reagenzstreifen :

Reagenzstreifen I dessen Aufbau und Abmessung in Beispiel 2 beschrieben ist und der in einem Testbezirk 1,9 mg MBTHS und 0,64 mg $K_4[Fe(CN)_6]$ und in einem weiteren Bezirk 30 U Sarkosinoxidase, 300 U Creatinase und 30 U Peroxidase enthält

Reagenzstreifen II (Abmessung 75 × 6 mm) mit einem Testbezirk 6 × 6 mm welcher 30 U Creatininase enthält.

Herstellung der Reagenzlösung :

Der Reagenzstreifen I wird 5-10 Sekunden lang in 32 ml der Pufferlösung bewegt, anschließend wird 5 Minuten stehen gelassen, nochmals 5-10 Sekunden bewegt und der eluierte Reagenzträger verworfen.

Bestimmungsansatz :

2 ml der Reagenzlösung werden mit 0,05 ml Probe versetzt. Nach 10 Minuten Inkubation bei 20-25 °C wird die Extinktion ($E_1$) bei 578 nm ermittelt (endogenes Kreatin).

Anschließend wird Reagenzstreifen II in analoger Weise in dieser Lösung eluiert und die Extinktion ($E_2$) nach 25 Minuten Inkubation bei 20-25 °C bei 578 nm gemessen.

Parallel dazu wird unter gleichen Bedingungen ein Creatinin-Standard mitgeführt. Die Konzentration (C) wird berechnet nach

$$C[\text{mg}/100\ \text{ml}] = \frac{\Delta E\ \text{Probe}}{\Delta E\ \text{Standard}}$$

$$\Delta E = E_2 - E_1$$

## Beispiel 4

Lagerstabilität von Substraten

1 mmolare Lösungen der Substrate N-Ethyl-N-ß-hydroxiethyl-m-toluidin (EHT) und N-Ethyl-N-ßsulfophenylenmethyl-m-toluidin (ESPT) in Phosphatpuffer pH 7,0 wurden 3 Wochen lang bei verschiedenen Temperaturen belastet und die Konzentration per HPLC bestimmt

| Substrat | Wochen Belastung | Wiederfindung in % bei 35° C | 50° C | 60° C |
|---|---|---|---|---|
| EHT | 1 | - | 87 % | 78 % |
| | 2 | - | 86 % | 76 % |
| | 3 | 89 % | 83 % | 68 % |
| ESPT | 1 | - | 99 % | 100 % |
| | 2 | - | 100 % | 99 % |
| | 3 | 99 % | 101 % | 100 % |

## Beispiel 5

Nachweis von Glucose im Urin

Filterpapier (Whatman Nr. 6) wird mit einer Lösung folgender Zusammensetzung imprägniert und bei 40-60 °C getrocknet :

| | |
|---|---|
| Glucoseoxidase | 200 mg |
| Peroxidase | 20 mg |
| Morpholino-äthansulfonat (1 molar, pH 6) | 65 ml |
| ECPT | 200 mg |
| MBTHS | 200 mg |
| Polyvinylpyrrolidon | 300 mg |
| Äthanol | 30 ml |
| Wasser dest. | ad 100 ml |

Das Testpapier reagiert mit Urinen die von 10-100 mg/dl Glucose enthalten mit hellrosa-violett bis dunkelblau-violetten Farbtönen.

Bestimmung von Peroxidase

Reagenzlösungen

Lösung 1

| | |
|---|---|
| S-MBTH | 3,2 mmol/l |
| Natriumlaurylsulfat | 1 g/l |
| 3,3-Dimethylglutarsäure/NaOH-Puffer pH 6,6 | 50 mmol/l |
| Wasser als Lösungsmittel | |

Lösung 2

| | |
|---|---|
| ETTS | 10 mmol/l |
| Natriumperborat | 480 mmol/l |
| EDTA | 400 mmol/l |
| Dimethylglutarsäurepuffer pH 6,6 | 50 mmol/l |
| Wasser als Lösungsmittel | |

Je 1 ml der Lösungen 1 und 2 werden mit 20 µl Probe gemischt und nach 5 Minuten die Absorption bei 588 nm gegen einen Reagenzleerwert gemessen. Die Konzentration wird durch Vergleich mit der Absorption bekannter Konzentrationen ermittelt. Die folgende Tabelle enthält solche Meßwerte.

| POD/l Reagenzlösung | Δ E (E Probe - E Leerwert) |
|---|---|
| 1ooo ng | 772 mE |
| 75o ng | 585 mE |
| 5oo ng | 381 mE |
| 25o ng | 2o1 mE |
| 125 ng | 1oo mE |
| 5o ng | 42 mE |
| 25 ng | 25 mE |
| o ng | o mE |

**Patentansprüche**

1. Mittel zum Nachweis von
a) Wasserstoffperoxid beziehungsweise Wasserstoffperoxid bildenden Substraten, oder
b) Peroxidasen oder Substanzen, die peroxidatisch wirken, bestehend aus einem kupplungsfähigen Substrat und einer für die Trinder-Reaktion bekannten oxidationsfähigen Substanz, sowie gegebenenfalls anderen, für die Trinder-Reaktion bekannten Zusatzstoffen und
a) einer Peroxidase oder
b) $H_2O_2$ und $H_2O_2$-bildenden Substanz, dadurch gekennzeichnet, daß als kupplungsfähiges Substrat eine Verbindung der allgemeinen Formel I

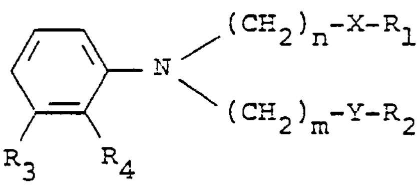

(I)

in der

n und m eine ganze Zahl von 1-4,

X und Y einen Valenzstrich oder einen Phenylenrest,

$R_1$ und $R_2$ eine Carboxyl-, oder Sulfonsäuregruppe darstellen, wobei eine der beiden Gruppen auch Wasserstoff oder eine niedere Alkylgruppe sein kann und

$R_3$ und $R_4$ Wasserstoff oder eine Alkylgruppe mit 1-6 vorzugsweise 1-2 C-Atomen darstellt sowie ihre Salze und als oxidationsfähige Substanz 2-Hydrazono-2,3-dihydro-3-methylbenzthiazol-sulfosäure-6 (S-MBTH) enthalten sind.

2. Mittel zum Nachweis von

a) Wasserstoffperoxid beziehungsweise Wasserstoffperoxid bildenden Substraten, oder

b) Peroxidasen oder Substraten, die peroxidatisch wirken, bestehend aus einem kupplungsfähigen Substrat und einer für die Trinder-Reaktion bekannten oxidationsfähigen Substanz, sowie gegebenfalls Zusatzstoffen und

a) einer Peroxidase oder

b) $H_2O_2$ und $H_2O_2$ -bildenden Substanz, dadurch gekennzeichnet, daß als kupplungsfähiges Substrat eine Verbindung der allgemeinen Formel I

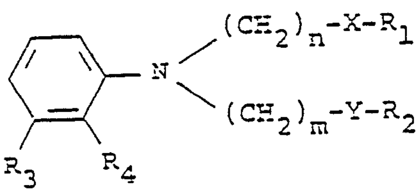

in der

n und m eine ganze Zahl von 1-4,

X und Y einen Valenzstrich oder einen Phenylenrest,

$R_1$ und $R_2$ eine Carboxyl- oder Sulfonsäuregruppe darstellen, wobei eine der beiden Gruppen auch Wasserstoff oder eine niedere Alkylgruppe sein kann und

$R_3$ und $R_4$ Wasserstoff oder eine Alkylgruppe mit 1-6, vorzugsweise 1-2 C-Atomen darstellt sowie ihre Salze, wobei $R_3$ eine Alkylgruppe darstellt wenn X-$R_1$ oder Y-$R_2$ eine Sulfo-Phenylengruppe darstellt.

3. Mittel gemäß Anspruch 1 oder 2, dadurch gekennzeichnet, daß Reagenzien, insbesondere eine Substratoxidase enthalten sind, die mit dem Substrat Wasserstoffperoxid bilden.

4. Mittel gemäß Ansprüchen 1 bis 3, dadurch gekennzeichnet, daß die Reagenzien auf einen saugfähigen Träger imprägniert sind.

5. Verfahren zum Nachweis von Wasserstoffperoxid beziehungsweise Wasserstoffperoxid bildenden Substraten oder Peroxidasen; dadurch gekennzeichnet, daß man ein Mittel gemäß einem der Ansprüche 1 bis 4 mit der Probe zusammenbringt und die erhaltene Verfärbung visuell oder mit einem Photometer auswertet.

6. Verfahren nach Anspruch 5, dadurch gekennzeichnet, daß man ein Mittel nach Anspruch 4 kurz in die Probe eintaucht und die Verfärbung in bekannter Weise bestimmt.

7. Verfahren nach Anspruch 5, dadurch gekennzeichnet, daß man ein Mittel nach Anspruch 4 solange in die Probe eintaucht, bis die Reagenzien eluiert sind und die Verfärbung gegebenenfalls nach Verdünnen oder Durchleiten von Sauerstoff mit einem Photometer bestimmt.

**Claims**

1. Agent for the detection of a) hydrogen peroxide or of hydrogen peroxide-forming substrates or b) peroxidases or substances which act peroxidatively, consisting of a couplable substrate and of an oxidisable substance known for the Trinder reaction, as well as possibly other additional materials known for the Trinder reaction, and a) of a peroxidase or b) $H_2O_2$ or $H_2O_2$-forming substance, characterised in that it contains, as couplable substrate, a compound of the general formula I

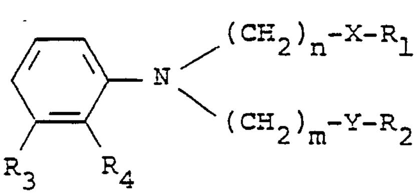

(I)

in which

n and m represent an integer of 1-4,

X and Y a valency bond or a phenylene residue

$R_1$ and $R_2$ a carboxylic or sulphonic acid group, whereby one of the two groups can also be hydrogen or a lower alkyl group and

$R_3$ and $R_4$ represent hydrogen or an alkyl group with 1-6, preferably 1-2 C-atoms, as well as their salts, and, as oxidisable substance, 2-hydrozono-2,3-dihydro-3-methylbenzthiazole-6-sulphonic acid (S-MBTH).

2. Agent for the detection of a) hydrogen peroxide or of hydrogen peroxide-forming substrates or b) peroxidases or substrates which act peroxidatively, consisting of a coupable substrate and of an oxidisable substance known for the Trinder reaction, as well as possibly additional materials and a) of a peroxidase or b) $H_2O_2$ or $H_2O_2$-forming substance, characterised in that, as coupable substrate, it contains a compound of the general formula I

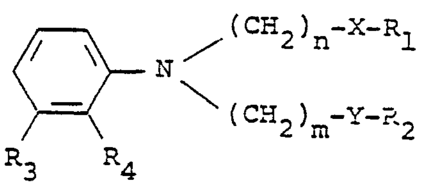

in which

n and m represent an integer of 1-4,

X and Y a valency bond or a phenylene residue

$R_1$ and $R_2$ a carboxylic or sulphonic acid group, whereby one of the two groups can also be hydrogen or a lower alkyl group and

$R_3$ and $R_4$ represent hydrogen or an alkyl group with 1-6, preferably 1-2 C-atoms, as well as their salts, whereby $R_3$ represents an alkyl group when X-$R_1$ or Y-$R_2$ represents a sulphophenylene group.

3. Agent according to claim 1 or 2, characterised in that reagents, especially a substrate oxidase, are contained which form hydrogen peroxide with the substrate.

4. Agent according to claims 1 to 3, characterised in that the reagents are impregnated on to an absorbent carrier.

5. Process for the detection of hydrogen peroxide or of hydrogen peroxide-forming substrates or peroxidases, characterised in that one brings an agent according to one of claims 1 to 4 together with the sample and evaluates the coloration obtained visually or with a photometer.

6. Process according to claim 5, characterised in that one briefly dips an agent according to claim 4 into the sample and determines the coloration in known manner.

7. Process according to claim 5, characterised in that one dips an agent according to claim 4 so long into the sample that the reagents are eluted and determines the coloration with a photometer, possibly after dilution or passing through of oxygen.

**Revendications**

1. Produit permettant de déceler la présence

a) d'eau oxygénée ou de substrats donnant de l'eau oxygénée ou

b) de peroxydases ou de substances agissant comme des peroxydases, et constitué par un substrat copulable et par une substance oxydable connue pour la réaction de Trinder ainsi que, le cas échéant, par des produits d'addition connus pour la réaction de Trinder et

a) par une peroxydase ou

b) par $H_2O_2$ ou une substance donnant $H_2O_2$, caractérisé en ce qu'il contient, comme substrat copulable, un composé ayant comme formule générale I

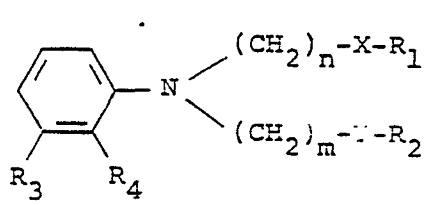

dans laquelle

n et m sont des nombres entiers compris entre 1 et 4

X et Y un trait de valence ou un reste phényle

$R_1$ et $R_2$ représentent un groupe carboxyle ou un groupe acide sulfonique, l'un des deux groupes pouvant être également de l'hydrogène ou un groupe alkyle inférieur, et

$R_3$ et $R_4$ représentent de l'hydrogène ou un groupe alkyle contenant de 1 à 6 atomes de carbone, de

préférence de 1 à 2 atomes de carbone, ainsi que leurs sels et, comme substance oxydable, l'acide 6-sulfonique du 2-hydrazone 2,3-dihydro 3-méthylbenzothiazole (S-MBTH).

2. Produit permettant de déceler la présence

a) d'eau oxygénée ou de substrats donnant de l'eau oxygénée, ou de

b) peroxydases ou de substrats agissant comme des peroxydases, et constitué par un substrat copulable et par une substance oxydable connue pour la réaction de Trinder ainsi que, le cas échéant, par des produits d'addition et

a) par une peroxydase, ou

b) par $H_2O_2$ ou une substance donnant $H_2O_2$ caractérisé en ce qu'il contient, comme substrat copulable, un composé ayant comme formule générale I

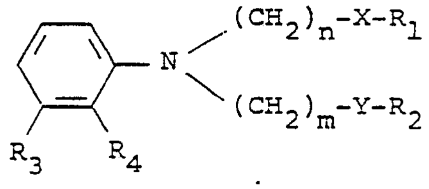

dans laquelle

n et m sont des nombres entiers compris entre 1 et 4

X et Y, un trait de valence ou un reste phényle

$R_1$ et $R_2$ représentent un groupe carboxyle ou un groupe acide sulfonique, l'un des deux groupes pouvant être également de l'hydrogène ou un groupe alkyle inférieur et

$R_3$ et $R_4$ représentent de l'hydrogène ou un groupe alkyle contenant de 1 à 6, de préférence 1 ou 2 atomes de carbone, ainsi que leurs sels, $R_3$ représentant un groupe alkyle lorsque $X-R_1$ ou $Y-R_2$ représente un groupe sulfo-phényle.

3. Produit selon l'une des revendications 1 ou 2, caractérisé en ce qu'il contient des réactifs, notamment une oxydase du substrat, qui, avec le substrat, donnent de l'eau oxygénée.

4. Produit selon l'une des revendications 1 à 3, caractérisé en ce que les réactifs imprègnent un support absorbant.

5. Procédé de détection d'eau oxygénée ou de substrats donnant de l'eau oxygénée ou de peroxydases, caractérisé en ce que l'on met en présence de l'échantillon un produit selon les revendications 1 à 4 et que la coloration obtenue est évaluée à l'œil nu ou au moyen d'un photomètre.

6. Procédé selon la revendication 5, caractérisé en ce que l'on plonge brièvement dans l'échantillon un produit selon la revendication 4 et que l'on détermine la coloration d'une manière connue.

7. Procédé selon la revendication 5, caractérisé en ce que l'on plonge dans l'échantillon un produit selon la revendication 4 jusqu'à ce que les réactifs soient élués, la coloration étant déterminée, le cas échéant après dilution ou passage d'oxygène, au moyen d'un photomètre.